# EUROPEAN PATENT SPECIFICATION

(11) **EP 2 570 152 B1**
(45) Date of publication and mention of the grant of the patent: **22.08.2018**
(21) Application number: 11181696.3
(22) Date of filing: 16.09.2011
(51) Int. Cl.: A61N 1/05

(54) **Structured probes for neural applications**
Strukturierte Sonden für neurale Anwendungen
Sondes structurées pour applications neuronales

(43) Date of publication of application: 20.03.2013
(73) Proprietor: IMEC VZW, 3001 Leuven (BE); Albert-Ludwigs-Universität Freiburg, 79098 Freiburg (DE)
(72) Inventor: Pereira Neves, Hercules, B-1320 Hamme-Mille (BE); Ruther, Patrick, D-76189 Karlsruhe (DE); Herwik, Stanislav, D-79618 Rheinfelden (DE); Torfs, Tom, B-1950 Kraainem (BE); Aarts, Arno, 3000 Leuven (BE)
(74) Representative: AWA Sweden AB

(56) References cited:
- WO-A1-2011/067297
- DE-A1-102010 000 565
- US-A1- 2010 331 935

## Description

### FIELD OF THE INVENTION

The present invention relates to a probe for brain recording or stimulation and a method for fabricating the same.

### BACKGROUND TO THE INVENTION

The recording or stimulation of neurons helps understanding how the brain processes information and controls bodily functions. In order to enable the recording or stimulation of the brain, a neural microelectrode (also called neural probe) can be inserted into a brain until the tip reaches the region of the brain to be stimulated/recorded.

A major criterion for the neural microelectrode design is to have as minimal a footprint as possible to minimize damage to the brain tissue and neuron network. Typically, a shank thickness of less than 50 µm for silicon-based probes is preferred. When multisite elongated neural microelectrode structures (e.g. with lengths longer than 6 mm) are desired, the design and fabrication of the microelectrodes is further complicated. The electrodes should have the ability to withstand the different types of forces during the insertion phase of the surgical implantation of the probe. The main forces that act upon the electrode probe during handling and insertion into the brain tissue are: the bending force which is a result of the out-of-plane loading causing parallel displacement to the tissue plane, the buckling force which occurs under axial compression as a result of the force that counteracts the normal force exerted on the brain tissue by the probe, and the shear force that prevents the tip of the probe from slipping on the surface of the brain tissue.

As one wants to make the neural probes as thin as possible the fragility of the probe also increases, particularly when relatively brittle materials such as silicon are used.

US2009012593 discloses a multiple-electrode probe for deep brain stimulation whereby reinforcements are used. The neural probe comprises longitudinal beam structures made of a biocompatible metal or carbon fiber. U.S. Pat. N°5,855,801 discloses a micro needle fabricated by using heavy boron doping to define the shape of the probe cross-section. The probe is sequentially reinforced by thickening the probe by leaving undoped silicon on the probe. Such a heavy boron doping is typically incompatible with the integration of electronics on the shaft itself. US2010/0331935 discloses a thin film spine-reinforced microelectrode array probe for analyzing neuronal activity, has elongated rigid spine fixedly connected with probe body using bio-adhesive material to structurally reinforce probe body. A need still exists for probes for brain recording or stimulation which can pierce through the brain membrane (also called Dura mater) without causing harmful dimpling of the Dura (e.g. which comprise a small cross-section) whilst providing a solution for the problem of bending and buckling. There is also still a need for a method for fabricating the same.

### SUMMARY OF THE INVENTION

It is an object of the present invention to provide a probe for brain recording or stimulation and a method for fabricating the same.

This object is met with the means according to the independent claims of the present invention. The dependent claims relate to preferred embodiments.

In a first aspect, the present invention relates to a method for manufacturing a probe for brain recording and/or stimulation, said probe comprising one or more shanks having a same orientation, wherein each of said shanks has a first side and a second side opposed to said first side and comprises at least one recording and/or stimulating site on said first side and a fin (or fins) protruding perpendicularly from said second side and running on at least a part of the length of said shank along said same orientation, wherein each of said shanks has a sharp pointed tip at its distal end, said tip lying in the plane defined by said first side. In an embodiment, the method according to the invention comprises the steps of:
a1) providing a substrate having a first surface and a second surface opposed to said first surface, said first surface comprising said at least one recording and/or stimulating site of each of said one or more shanks of the probe to be manufactured, and
a2) removing part of said substrate at the second side or attaching a fin (or fins) to said second side so as to form a substrate having a fin (or fins) protruding perpendicularly from said second side and running on at least a part of the length of said shank to be manufactured along said same orientation, and further comprises the steps of:
   a1) providing a substrate having a first and a second surface opposed to said first surface, said first surface comprising said at least one recording and/or stimulating site of each of said one or more shanks of the probe to be manufactured,
   a2(i) providing a first etch mask pattern on said second surface, said first etch mask pattern masking the area corresponding to the fin of each of said one or more shanks of the probe to be manufactured, said first etch mask pattern being resistant to an etching process, and
   a2(ii) etching said second surface with a etching process for which said first etch mask is resistant, in a direction perpendicular to said second surface in such a way as thinning, but not eliminating, at least part of said substrate away from said first etch mask pattern, thereby defining the height of said fins, and
b) sharpening a distal end of said shanks in such a way as to provide said shanks with a sharp pointed tip at its distal end, said tip lying in the plane defined by said first side.

As used herein and unless provided otherwise, the term "etching" encompass both dry-etching and wet-etching but dry-etching is advantageous due to its anisotropic character. Wet-etching can be made anisotropic e.g. by using a (110) Si substrate.

The method according to the invention is advantageous as it permits the production of thin shanks having a rigidity in the direction perpendicular to the surface of their first or second side which is similar to the rigidity of thicker shanks. This rigidity is about the same as the rigidity of a shank not having said fin but having a thickness equal to the sum of the thickness of the shank and the height of the fin. The shanks of the present invention are therefore rigid enough to penetrate the brain without bending while being thin enough to minimize damage to the brain.

Preferably, the method provides said shank(s) with a T-shaped cross-section.

In a preferred embodiment of the first aspect, the present invention relates to a method for manufacturing a probe for brain recording and/or stimulation, said probe comprising a connector part from which one or more shanks protrude along a same orientation, wherein each of said shanks has a first side and a second side opposed to said first side and comprises at least one recording and/or stimulating site on said first side and a fin (or fins) protruding perpendicularly from said second side and running on at least a part of the length of said shank along said same orientation, thereby preferably providing said shank with a T-shaped cross-section, the method comprising the steps of:
a1. providing a substrate having a first and a second surface opposed to said first surface, said first surface comprising said at least one recording and/or stimulating site of each of said one or more shanks of the probe to be manufactured,
S. Providing on said second surface, a second etch mask pattern delimiting the shape of the shanks and masking the area corresponding to said connector part of the probe to be manufactured, said second etch mask pattern being resistant to an etching process,
a2(i). providing a first etch mask pattern on said second surface, said first etch mask pattern masking exactly the area corresponding to the fin(s) of each of said one or more shanks and the area corresponding to said connector part of the probe to be manufactured by (preferably exactly) covering said second etch mask pattern, said first etch mask pattern being resistant to an etching process,
a2(ii). etching said second surface with an etching process for which said first etch mask pattern is resistant, in a direction perpendicular to said second surface in such a way as thinning, but not eliminating, at least part of said substrate away from said first etch mask pattern, thereby defining the height of said fin(s),
a2(iii) removing said first etch mask pattern without removing said second etch mask pattern,
a2(iv) etching said second surface with an etching process for which said second etch mask pattern is resistant, in a direction perpendicular to said second surface in such a way as thinning but not eliminating at least part of said substrate away from said second etch mask pattern, thereby defining the thickness of said shanks, and
a3. at least partially cutting out said probe from said substrate.

In a second aspect, the present invention relates to a probe for brain recording and/or stimulation, said probe comprising one or more shanks having a same orientation, wherein each of said shanks has a first side and a second side opposed to said first side and comprises at least one recording and/or stimulating site on said first side and a fin (or fins) protruding perpendicularly from said second side and running on at least a part of the length of said shank along said same orientation, wherein each of said shanks has a sharp pointed tip at its distal end, said tip lying in the plane defined by said first side. In a preferred embodiment, said fins are thereby providing said shank with a T-shaped cross-section. In other words, in a preferred embodiment, said probe may comprise shanks having a T-shaped profile, said T-shape being defined by a horizontal part and a vertical part, wherein said horizontal part has a first side and a second side, wherein said vertical part is attached to said second side, and wherein said first surface comprises one or more stimulating and/or recording sites (e.g. electrodes).

An advantage of embodiments of the present invention is that the second moment of inertia of the shanks of the probe is increased while maintaining a total cross-sectional area as small as possible.

In an embodiment, a probe for brain recording and/or stimulation comprises one or more shanks having a same orientation, wherein each of said shanks has a first side and a second side opposed to said first side and comprises at least one recording and/or stimulating site on said first side and a fin protruding perpendicularly from said second side and running on at least a part of the length of said shank along said same orientation, each of said shanks having a sharp pointed tip at its distal end, said tip lying in the plane defined by said first side.

In a preferred embodiment, the probe may comprise at least one shank being a first shank according to any embodiment of the second aspect, which fin is further attached to either:
a) the fin of a second shank as described in any embodiment of the first aspect, or
b) the second side of a second shank having a first side and a second side opposed to said first side and comprising at least one recording and/or stimulating site on said first side, or
c) the second side of a second shank having a first side and a second side opposed to said first side,
thereby providing a probe having two parallel sets of shanks, wherein each shank in the first set is linked to a shank in the second set via said fin. In an embodiment, this further attachment of the fin may provide a H- shaped profile to said shank. In an embodiment, the fin of said second shank may belong to a second probe for brain recording and/or stimulation, comprising one or more shanks having said same orientation.

Such an H-shaped profile further rigidifies the shank and provides (in cases (a) and (b) two surfaces for recording and/or stimulating. Such an H-shaped shank is easier to insert in a brain than a flat shank of total thickness lower than the total thickness of the H-shaped profile. The reason is that in the H-shaped profile, each of the two shanks composing the H-shape can be thinner than the flat shank, even if the total structure is thicker. The fin being relatively thin, it does not interfere much with the penetration of the H-shaped shank. In an embodiment, the distance between two adjacent shanks in the first set is equal to the distance between two adjacent shanks in the second set.

In a third aspect, the present disclosure relates to a method of fabricating a probe for brain recording or stimulation, said probe having two parallel sets of shanks (e.g. comprising shanks having a H-shaped cross-section), the method comprising the step of attaching the free extremity of the fin of a shank of a probe according to any embodiment of the second aspect to either:
a) the fin of a second shank according to any embodiment of the second aspect, or
b) the second side of a second shank having a first side and a second side opposed to said first side and comprising at least one recording and/or stimulating site on said first side, or
c) the second side of a second shank having a first side and a second side opposed to said first side.

In an example, the fin of said second shank may belong to a second probe for brain recording and/or stimulation, comprising one or more shanks having said same orientation.

In a fourth aspect, the present disclosure relates to a probe comprising one or more shanks having a same orientation, wherein each of said shanks has a first side and a second side opposed to said first side and comprises at least one recording and/or stimulating site on said first side which second side is attached to the second side of a second shank having a first side and a second side opposed to said first side and comprising at least one recording and/or stimulating site on said first side, thereby providing a probe having two parallel sets of shanks, wherein each shank in the first set is linked to a shank in the second set. In an embodiment, the distance between two adjacent shanks in the first set is equal to the distance between two adjacent shanks in the second set.

Although there has been constant improvement, change and evolution of devices in this field and methods for fabricating the same, the present concepts are believed to represent departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only. The scope of the invention is defined by the independent claims. Peferred embodiments are given in the dependent claims. The reference figures quoted below refer to the attached drawings.

### BRIEF DESCRIPTION OF THE DRAWINGS

Further features of the present invention will become apparent from the drawings, wherein:
FIG. 1 is a schematic representation of a probe according to an embodiment of the second aspect of the present invention.
FIG. 2 is a schematic representation of a method according to an embodiment of the first aspect of the present invention. In this representation, a cross-section orthogonal to the probe shank through one electrode is represented.
Fig. 3 is a schematic representation of a method according to an embodiment of the first aspect of the present invention. In this representation, a cross-section along a shank is represented. The fin fabrication is not represented in this figure.
Fig. 4 is a picture of a probe according to an embodiment of the present invention and showing the second side (backside) of said probe.
Fig. 5 is a picture of a probe according to an embodiment of the present invention and showing the first side (front side) of the shanks.
Fig. 6 is a picture of the second side of the distal end of a shank in a probe according to an embodiment of the present invention.
Fig. 7 is a picture of the first side of the distal end of a shank in a probe according to an embodiment of the present invention.
Fig. 8 is a schematic representation of a probe according to an embodiment of the second aspect of the present invention. A sharpened shank is represented but the fin is not shown.
Fig. 9a is a schematic perspective view of a shank having a T-shaped cross-section according to an embodiment of the present invention.
Fig. 9b is a schematic perspective view of a shank having a H-shaped cross-section according to an embodiment of the present invention.
Fig. 10 illustrates a probe array obtainable by an embodiment of the invention. The fins are not represented.
Fig. 11 shows a carrier block usable in the method of the invention, and an attachment location of a probe to said carrier block.
Fig. 12 shows a probe attached to a carrier block.
Fig. 13 shows a rotating grinding tool being used in conjunction with the carrier block and a probe attached thereto.
Fig. 14 shows the shape of the shanks distal ends before and after grinding, according to one embodiment of a method of the invention.
Fig. 15 shows the shape of the shanks distal ends before and after grinding, according to another embodiment of a method of the invention.
Fig. 16 shows an example of the dimensions of a carrier block usable in a method of the invention.

### DETAILED DESCRIPTION OF EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. Embodiments and aspects listed below do not, insofar as they do not comprise all features of the appended independent claims, and in particular insofar as they do not comprise the characterising part of the appended independent claims, form part of the claimed invention and are of exemplary nature. In a first aspect, the present invention relates to a method for manufacturing a probe for brain recording and/or stimulation, said probe comprising one or more shanks having a same orientation, wherein each of said shanks has a first side and a second side opposed to said first side and comprises at least one recording and/or stimulating site on said first side and a fin protruding perpendicularly from said second side and running on at least a part of the length of said shank along said same orientation, preferably thereby providing said shank with a T-shaped cross-section.

In an embodiment, the method may comprise the steps of:
a1) providing a substrate having a first surface and a second surface opposed to said first surface, said first surface comprising said at least one recording and/or stimulating site of each of said one or more shanks of the probe to be manufactured, and
a2) removing part of said substrate at the second side or attaching a fin to said second side so as to form a substrate having a fin protruding perpendicularly from said second side and running on at least a part of the length of said shank to be manufactured along said same orientation.

In another embodiment, the method may further comprise the step (a3) of cutting out at least partially said probe from said substrate and the step (b) of sharpening the distal end of said shanks in such a way as to provide said shanks with a sharp pointed tip at their distal end, said tip lying in the plane defined by said first side.

In another embodiment, the method may comprise the steps of:
a) providing a probe for brain recording and/or stimulation, said probe comprising one or more shanks having a same orientation, wherein each of said shanks has a first side and a second side opposed to said first side and comprises at least one recording and/or stimulating site on said first side and a fin protruding perpendicularly from said second side and running on at least a part of the length of said shank along said same orientation, and
b) sharpening the distal end of said shanks in such a way as to provide said shanks with a sharp pointed tip at its distal end, said tip lying in the plane defined by said first side.

In an embodiment, said sharpening may comprise the steps of
b1) providing a carrier having a first and second flat outer surface oriented at an angle to each other, so that said surfaces are joined by an edge of said carrier,
b2) attaching the shanks to the second surface of the carrier, with the second side of the shanks facing said carrier, the distal end being in close proximity to said edge, and
b3) subjecting the first surface of the carrier and at least part of the distal end of the second side of the probe to a grinding step, to thereby remove material at least from said distal end of the probe.

In step (b2), the shanks may be attached via their fins or via other means. In an embodiment, a major portion of the shanks may be attached (e.g. via a major portion of the fins) to said second surface. A major portion may mean more than 50% of the length, preferably more than 90% of the length.

In another embodiment, step (a2) may comprise the steps of
i) providing a first etch mask pattern on said second surface, said first etch mask pattern masking the area corresponding to the fin of each of said one or more shanks of the probe to be manufactured, said first etch mask pattern being resistant to an etching process, and
ii) etching said second surface with an etching process for which said first etch mask pattern is resistant, in a direction perpendicular to said second surface in such a way as thinning, but not eliminating, at least part of said substrate away from said first etch mask pattern, thereby defining the height of said fins.

Therefore, in an embodiment of the present invention, the method (or step (a) of the method) may comprise the steps of:
a1) providing a substrate having a first and a second surface opposed to said first surface, said first surface comprising said at least one recording and/or stimulating site of each of said one or more shanks of the probe to be manufactured,
a2(i)) providing a first etch mask pattern on said second surface, said first etch mask pattern masking the area corresponding to the fin of each of said one or more shanks of the probe to be manufactured, said first etch mask pattern being resistant to an etching process, and
a2(ii)) etching said second surface with an etching process for which said first etch mask is resistant, in a direction perpendicular to said second surface in such a way as thinning, but not eliminating, at least part of said substrate away from said first etch mask pattern, thereby defining the height of said fins.

This is advantageous as it permit the production of thin shanks having a rigidity in the direction perpendicular to the surface of their first or second side which is similar to the rigidity of thicker shanks. This rigidity is about the same as the rigidity of a shank not having said fin but having a thickness equal to the sum of the thickness of the shank according to the present embodiment and the height of the fin. The shanks of the present invention are therefore rigid enough to penetrate the brain without bending while being thin enough to minimize damage to the brain.

Step (a1) may involve providing a naked substrate, optionally providing a mask layer on this substrate, providing a (patterned) metal layer on said mask and passivating said metal layer, thereby defining said stimulating/recording sites and the corresponding leads.

The metal layer can for instance be deposited by physical vapour deposition, electron beam physical vapour deposition, thermoevaporation, sputtering or electroplating amongst others. If the electrodes/sites are in IrO, electroplating can be used to grow IrO on Pt or sputtering can be used in an oxygen containing atmosphere.

In an embodiment of the first (and the second) aspect, said probe may further comprise a connector part from which said one or more shanks protrude along said same orientation. In this embodiment of the first aspect, in step (a2(i)), said first etch mask pattern may further mask the area corresponding to said connector part of the probe to be manufactured. This is advantageous because it prevents the connector to be thinned when the shanks are thinned and the height of the fins is defined.

In an embodiment of the first aspect, the first etch mask pattern may further be delimiting the shape of the shanks of the probe to be fabricated. This permits to thin the part of the substrate corresponding to the shanks to be manufactured, thereby defining said shanks.

In an embodiment, step (a2(i)) may comprise providing on said second surface, a layer covering the orthogonal projection on said second surface of the shape of the probe to be fabricated, said layer being resistant to said etching process, and patterning said layer.

The layer deposited in step (a2(i)) may for instance be deposited by chemical vapour deposition.

In an embodiment of the first aspect and of the second aspect, said fin may be running in the middle of said second surface of said shank so as to divide longitudinally said second surface in two equal parts. This geometry maximizes the rigidity of the shanks.

In an embodiment, said fin may be running on at least 50%, preferably at least 75%, more preferably at least 85% of the length of said shank along said same orientation

Said first etch mask pattern is preferably masking exactly the area corresponding to the fin of each of said one or more shanks of the probe to be manufactured. This permits the definition of the fins during step (a2(ii)).

Step (a2(ii)) is preferably performed by an anisotropic etching method such as but not limited to reactive ion etching, chemistry enhanced reactive ion etching, magnetron enhanced reactive ion etching, plasma etching and the likes.

In an embodiment, the method according to the first aspect may further comprises a step (s) after step (a1) and before step (a2(ii)), of providing on said second surface, a second etch mask pattern delimiting the shape of the shanks of the probe to be fabricated, said second etch mask pattern being resistant to an etching process. This permits to thin the part of the substrate corresponding to the shanks to be manufactured, thereby defining said shanks.

Preferably, step (s) is performed before step (a2(i)). This permits the first etch mask pattern to be closer to the substrate in the area delimiting the shape of the shanks than the second etch mask pattern

In an embodiment of the first aspect, said second etch mask pattern may further mask the area corresponding to said connector part of the probe to be manufactured. This permits to prevent the connector part to be thinned when the first etch mask pattern is removed to enable the total thickness (including the fin) of the shanks to be reduced while keeping the height of the fin substantially constant.

In an embodiment of the first aspect, in step (a2(i)), in addition to masking said area corresponding to the fin, said first etch mask pattern may further mask the area corresponding to said connector part of the probe to be manufactured by covering said second etch mask pattern. Preferably, in addition to masking said area corresponding to the fin, said first etch mask pattern covers exactly said second etch mask pattern.

In an embodiment involving a step (s), step (a2(i)) may comprise providing a first etch mask pattern on said second surface, said first etch mask pattern masking exactly the area corresponding to the fin of each of said one or more shanks, masking the area corresponding to said connector part and delimiting the shape of the shank of the probe to be manufactured by (preferably exactly) covering said second etch mask pattern, said first etch mask pattern being resistant to an etching process.

In an embodiment, said first etch mask pattern and said second etch mask pattern have a different chemical nature in order to permit the selective removal of the first etch mask pattern. In an embodiment, the first etch mask pattern can be made of any photoresist (either positive or negative). In an embodiment, the first etch mask pattern can be made of any polymer. In another embodiment, the first etch mask pattern can be made of a metal (e.g. aluminum) that is compatible with the etching process. In an embodiment, the second etch mask pattern is preferably made of silicon oxide deposited using chemical vapor deposition. In another embodiment, it can be made of metal if compatible with the process.

In an embodiment of the first aspect involving a step (s), the method may further comprise after step (a2(ii)), a step of:
a2(iii). removing said first etch mask pattern without removing said second etch mask pattern, and
a2(iv). etching said second surface with said etching process for which said second etch mask pattern is resistant, in a direction perpendicular to said second surface in such a way as thinning but not eliminating at least part of said substrate away from said second etch mask pattern, thereby defining the thickness of said shanks.
a3. at least partially cutting out said probe from said substrate.

In this embodiment, step (a2(iv)) reduces the thickness of the shank without reducing the height of the fin.

In an embodiment, step (s) may comprise providing on said second surface, a layer covering the orthogonal projection on said second surface of the shape of the probe to be fabricated, said layer being resistant to said etching process, and patterning said layer by creating an opening in said layer, said opening comprising the orthogonal projection of the shanks of the probe to be fabricated.

The layer deposited in step (s) may for instance be deposited by chemical vapour deposition.

In an embodiment of the first aspect, a masking layer may be present on said first surface of said substrate below said at least one recording and/or stimulating site and wherein step (a3) is performed by patterning said masking layer so as to form openings in said masking layer around the shape of the probe to be fabricated and subsequently etching said first surface. This has the effect of etching through the first surface of the substrate wherever openings are present. If these openings are defining the complete contour of the probe to be fabricated, this etching sets the probe free from the rest of the substrate

In a preferred embodiment of the first aspect, the present invention relates to a method for manufacturing a probe for brain recording and/or stimulation, said probe comprising a connector part from which one or more shanks protrude along a same orientation, wherein each of said shanks has a first side and a second side opposed to said first side and comprises at least one recording and/or stimulating site on said first side and a fin protruding perpendicularly from said second side and running on at least a part of the length of said shank along said same orientation, thereby preferably providing said shank with a T-shaped cross-section, the method comprising the steps of:
(a1) providing a substrate having a first and a second surface opposed to said first surface, said first surface comprising said at least one recording and/or stimulating site of each of said one or more shanks of the probe to be manufactured,
(s) providing on said second surface, a second etch mask pattern delimiting the shape of the shanks and masking the area corresponding to said connector part of the probe to be manufactured, said second etch mask pattern being resistant to a etching process,
(a2(i)) providing a first etch mask pattern on said second surface, said first etch mask pattern masking exactly the area corresponding to the fin of each of said one or more shanks, masking the area corresponding to said connector part and delimiting the shape of the shank of the probe to be manufactured by (preferably exactly) covering said second etch mask pattern, said first etch mask pattern being resistant to an etching process,
(a2(ii)) etching said second surface with an etching process for which said first etch mask pattern is resistant, in a direction perpendicular to said second surface in such a way as thinning, but not eliminating, at least part of said substrate away from said first etch mask pattern, thereby defining the height of said fins,
(a2(iii)) removing said first etch mask pattern without removing said second etch mask pattern,
(a2(iv)) etching said second surface with an etching process for which said second etch mask pattern is resistant, in a direction perpendicular to said second surface in such a way as thinning but not eliminating at least part of said substrate away from said second etch mask pattern, thereby defining the thickness of said shanks, and
(a3) at least partially cutting out said probe from said substrate.

In an embodiment of the present invention, the patterning of said masking layer may be such that at least one attachment point between said probe and the substrate outside of said probe is maintained upon performing step (a2(iv)) and whereby said attachment point permits the detachment by hand of the probe from the rest of the substrate. This is the case where the openings are defining only part of the contour of the probe to be fabricated (i.e. portions of the contour are without opening in the masking layer). In this case, the etching only partly frees the probe from the rest of the substrate, i.e. attachment points remain between the probe and the rest of the substrate. These portions of the contour without opening in the masking layer are preferably present around the connecting part of the probe. The connecting part of the probe being less fragile than the shanks, there is less risk of breaking the probe upon manually detaching the probe from its substrate.

In an embodiment of the first aspect, the method may further comprise a step (b) of sharpening the distal end of said shanks. Shanks which are not sharpened at their distal end have difficulties in piercing through the brain membrane (also known as "Dura mater"). Sharpening the distal end of the shanks permits to avoid harmful dimpling of the dura mater upon implantation of the probe through the Dura mater.

Said sharpening is preferably performed by grinding the second side of said shanks. This technique may be performed by temporary fixing the unsharpened probe on a carrier (or jig) in such a way that the distal ends of the shanks are leading over the carrier. The carrier may be placed against a rotating disk and have thereby the second side of the shanks distal ends grinded under the same angle as the carrier.

Shanks sharpen by grinding of their second side are easy to implant through the Dura mater of a rhesus monkey. Since the Dura does not need to be opened, the risk of infection during surgery is lowered.

Other ways to sharpen the distal ends of the shanks are for instance using selective anisotropic etching techniques or wet etching techniques using high dopant implantation into a silicon substrate.

According to an embodiment of the method, a probe is provided, said probe comprising a connector part, and parallel shanks extending outward from said connector part, said shanks having an essentially constant thickness extending between a first and second side of the shank, and the fin protruding perpendicularly from said second side of said shank being attached to said second surface of the carrier, with the connector part and the majority of each shaft portion facing the second surface (e.g. attached via the fin or via other means), and the distal end (e.g. a triangular chisel shaped tip portions) being in close proximity to the edge, and wherein material is removed from the distal end of all of said shanks.

According to an embodiment, at least part of said distal end(s) is (are) extending outward from said edge, so that said part of the distal end(s) extend(s) below the first surface of the carrier when said first surface is held horizontally, and wherein substantially no material of the carrier is removed during said grinding step.

According to another embodiment, the shank(s) do(es) not extend outward from the edge, and wherein the carrier material and the shank tip material are removed simultaneously during said grinding step.

Said edge may be shaped as a straight line. Said shank(s) may be placed perpendicularly to said edge.

The location on the carrier block onto which the shank(s) is (are) attached may be provided with an alignment structure. Said alignment structure may comprise one or more grooves into which said shank or shanks may be placed. Said alignment structure may be a negative imprint of the probe. In an embodiment, said alignment structure may comprise on or more groves in which said fins may be placed.

According to an embodiment, the grinding step is done by placing the carrier on a rotating grinding disc and applying pressure on the carrier. The grinding step may be followed by a polishing step.

According to an embodiment, the grinding step is continued so as to obtain a sharp pointed tip, lying in the plane defined by the first side of the shank or probe.

According to another embodiment, the grinding step is followed by the steps of:
- attaching the first side of the probe to the second surface of the carrier, with the connector part and the majority of the shaft portion of each shank attached to said second surface and the distal end (tip portion) being in close proximity to said edge,
- subjecting the first surface of the carrier and at least the distal end (e.g. part of the tip portion) of the shank(s) to a grinding step, to thereby remove material at least from said distal end (e.g. a triangular, chisel shaped tip portion) of the probe(s), so as to obtain a sharp pointed tip lying in between the planes defined by the first and second side of the shank or probe.

In a second aspect, the present invention relates to a probe for brain recording and/or stimulation, said probe comprising one or more shanks having a same orientation, wherein each of said shanks has a first side and a second side opposed to said first side and comprises at least one recording and/or stimulating site on said first side and a fin protruding perpendicularly from said second side and running on at least a part of the length of said shank along said same orientation. Each shank comprises an elongate shaft portion and a tip portion. The tip portion is at the distal end of the shanks. The tip portion may have a triangular chisel-shape. The shafts have an essentially constant thickness extending between said first side and said second side.

In an embodiment of the second aspect, the shanks may have a T-shaped cross-section provided by said fin protruding perpendicularly from said second side.

In other words, the second aspect of the present invention may relate to a probe for brain recording or stimulation, said probe comprising shanks having a T-shaped profile, said T-shape being defined by a horizontal part and a vertical part, wherein said horizontal part has a first side and a second side, wherein said vertical part is attached to said second side, and wherein said first side comprises one or more recording and/or stimulating sites (e.g. electrodes).

In an embodiment of the second aspect, the probe may further comprise a connector part from which said one or more shanks protrude along said same orientation.

In an embodiment, there may be a shorter distance between said first and said second side of said shanks at the distal end of said shanks than away from said distal end. This sharpens the distal end of the shanks and helps to pierce through the Dura mater with a reduced dimpling effect.

In an embodiment, said first and said second surface of said shanks are not parallel at the distal end of said shanks. This sharpens the distal end of the shanks.

In an embodiment, said first and said second surface of said shanks may intersect in a line at the distal end of said shanks, said line producing an edge. This edge is thereby made sharp which permits it to cut through the dura mater without dimpling effect.

In an embodiment, said first side may have a same orientation at the distal end of the shank and away from said distal end while said second side at said distal end makes an angle with said second side away from said distal end. In other words, a sharp pointed tip may be present at the distal end of the shank, said pointed tip lying in the plane defined by the first side of the shank or probe.

In another embodiment, the present invention may relate to a probe for brain recording and/or stimulation, said probe comprising one or more shanks having a same orientation, wherein each of said shanks has a first side and a second side opposed to said first side and comprises at least one recording and/or stimulating site on said first side and a fin protruding perpendicularly from said second side and running on at least a part of the length of said shank along said same orientation, each of said shanks having a sharp pointed tip at its distal end, said tip lying in the plane defined by said first side.

This is the geometry obtained by grinding the second side of the shafts. This permits to avoid damages to the first side of the shafts (which comprise recording/stimulating sites) while sharpening the shafts. Such a sharpening would typically deviate the shanks from their trajectory upon insertion by inducing a bending upward (in the direction of the first side) of the shanks. However, this bending is efficiently prevented by the fin protruding perpendicularly from said second side of the shanks of the present invention. There is therefore synergy between the presence of the fin and the presence of the sharpening. An alternative way to prevent this bending would be to manufacture probes having shanks with a sharp pointed tip lying in between the planes defined by the first and second surface of the shank or probe. This would prevent bending in so far as the tip would be lying exactly at mid-distance between the planes defined by the first and second surface of the shank or probe. The manufacture of such tips is however delicate and requires two grinding steps while embodiments of the present invention permit to prevent bending even of tips obtained by only one grinding step.

In an embodiment, said shanks may have a triangular and/or chisel shaped tip at their distal end.

In an embodiment, each of said shanks may have a total length of 0.5 to 10 mm.

In an embodiment, said fin may have a rectangular parallelepiped shape.

In an embodiment, said shank may have a thickness (fin included) of from 15 to 100 µm, preferably from 15 to 50 µm, even more preferably from 15 to 30 µm and most preferably from 20 to 30 µm. In other words, this corresponds to the total height of the T-shape. This total height is preferably higher than 15 µm and more preferably higher than 20 µm in order to assure enough rigidity to the shanks. Below 15 µm, the bending tends to be too high.

In an embodiment, said shanks may have a thickness away from said fin (and from their distal end if the shanks are sharpened by reducing their thickness at their distal end) of from 1 to 20 µm, preferably 1 to 15 µm. Typically, when this thickness is 20 µm or lower, the rigidity of the shank (without fin) might be too low to assure proper penetration in the brain. In such a case, the presence of the fin is particularly advantageous.

In an embodiment, said fin may have a height of from 1 to 35 µm.

In an embodiment, said fin may have a width of from 1 to 30 µm, preferably from 5 to 20 µm.

In an embodiment, the width of said shanks away from their distal end may be from 40 µm to 200 µm, preferably from 40 µm to 140 µm, more preferably from 40 µm to 120 µm and even more preferably from 40 µm to 100 µm. The width of the shanks preferably is reduced (preferably to a point) at their distal end. This eases the penetration of the shanks in the brain.

In an embodiment, the width of said fin may be from 2 to 30 µm, preferably from 5 to 20 µm.

The area of a shanks's cross-section (e.g. T-shaped cross-section) is preferably smaller than 6000 µm², more preferably smaller than 2500 µm² and still more preferably smaller than 1250 µm² in order to minimize harm to the tissues. For instance this area can be from 500 to 1250 µm².

In an embodiment, the ratio between (i) the width of the shank away from its distal end and (ii) the thickness (fin included) of the shank can be from 1 to 3.5. Above 3.5, the bending of the shank tends to be too high.

In an embodiment, the probe may comprise at least one shank being a first shank according to any embodiment of the second aspect, which fin is further attached to either:
a) the fin of a second shank according to any embodiment of the first aspect, or
b) the second side of a second shank having a first side and a second side opposed to said first side and comprising at least one recording and/or stimulating site on said first side, or
c) the second side of a second shank having a first side and a second side opposed to said first side,
thereby providing a H- shaped profile to said shank.

In an embodiment, the fin of said second shank may belong to a second probe for brain recording and/or stimulation, comprising one or more shanks having said same orientation.

Such an H-shaped profile further rigidifies the shank and provides in cases (a) and (b) two surfaces for recording and/or stimulating. Such an H-shaped shank is easier to insert in a brain than a flat shank of total thickness lower than the total thickness of the H-shaped profile. The reason is that in the H-shaped profile, each of the two shanks composing the H-shape can be thinner than the flat shank, even if the total structure is thicker. The fin being relatively thin, it does not interfere much with the penetration of the H-shaped shank.

In a third aspect, the present invention relates to a method of fabricating a probe for brain recording or stimulation, said probe comprising a shank having a H-shaped cross-section, the method comprising the step of attaching the free extremity of the fin of a shank of a probe according to any embodiment of the second aspect to either:
a) the fin of a second shank according to any embodiment of the second aspect, or
b) the second side of a second shank having a first side and a second side opposed to said first side and comprising at least one recording and/or stimulating site on said first side, or
c) the second side of a second shank having a first side and a second side opposed to said first side.

In an embodiment, the fin of said second shank may belong to a second probe for brain recording and/or stimulation, comprising one or more shanks having said same orientation.

In a fourth aspect, the present invention relates to a probe comprising one or more shanks having a same orientation, wherein each of said shanks has a first side and a second side opposed to said first side and comprises at least one recording and/or stimulating site on said first side which second side is attached to the second side of a second shank having a first side and a second side opposed to said first side and comprising at least one recording and/or stimulating site on said first side, thereby providing a probe having two parallel sets of shanks, wherein each shank in the first set is linked to a shank in the second set. In an embodiment, the distance between two adjacent shanks in the first set is equal to the distance between two adjacent shanks in the second set.

In other words, in the fourth aspect, the probe comprises one or more composite shanks, each of said composite shank being formed of two component shanks attached back to back (second side to second side).

Such a probe has the advantage to have recording and/or stimulating sites on both its sides. Such a probe has the further advantage that the two component shanks have a tendency to bend in opposite directions of one another (due to the internal stress of the various thin film layers used in the probe fabrication), attaching them back to back cancels this tendency and gives rise to a surprisingly rigid probe. This is especially advantageous when said probe has integrated CMOS.

Such a probe has the further advantage that it can be made relatively thin. Each component shank may have a thickness of from 8 to 50 µm, preferably 8 to 25 µm and most preferably 8 to 15 µm. Therefore, the composite shanks may have a thickness of from 16 to 100 µm, preferably 16 to 50 µm and most preferably 16 to 30 µm. The length and the width away from the distal end can be as described for the second aspect of the present invention. An intermediate layer of a dissimilar material (for instance a metal or a polymer) can also be attached in between the two component shanks to modify the probe resistance to bending and/or breakage.

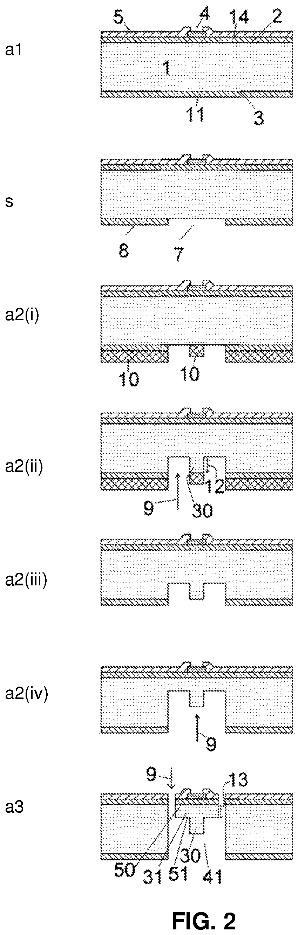

In an embodiment of the first aspect, the present invention relates to a method of fabricating a probe for brain recording or stimulation comprising shanks having a T-shaped cross-section defined by a vertical part 30 and a horizontal part 31 (illustrated orthogonal to the probe through one electrode in Fig. 2). The method comprises a step (a1) of providing a substrate 1 having a first 2 and a second 3 surface, whereby the first surface 2 comprises at least one electrode 4. In a next step (a2(i)) a first etch mask pattern 10 is provided on the second surface 3. Optionally, an isolation layer 14 is positioned directly under the electrode 4. The surface created by orthogonal projection of the electrode onto the second surface overlaps the first etch mask pattern 10. In step (a2(ii)) dry-etching of the second surface 3 is performed in a direction 9 perpendicular to the second surface 3 in such a way as thinning, but not eliminating, at least part of said substrate 1 away from the first etch mask pattern 10, thereby defining the height 12 of the vertical part 30 in said T-shaped cross-section.

In another embodiment, the method may further comprise a step (s) after step (a1) and before step (a2(ii)) of providing a second etch mask pattern 8 on the second surface 3, surrounding but not overlapping the area corresponding to the area of the orthogonal projection of the electrode 4, and whereby the second etch mask pattern 8 is resistant to a dry etching process. Preferably step (s) is performed before step (a2(i)).

Preferably after the electrodes are defined in the first surface 2 and before the first step (a1) of the method according to an embodiment of the invention, passivation can be applied on said first surfaces of the substrate. The substrate material used is preferably a semiconductor and more specifically silicon.

The electrode can be deposited using several deposition techniques known in the art, such as for example electron beam evaporation, sputter deposition or plasma vapour deposition or any other conventional thin film deposition technique.

In yet another embodiment, before step (s), a layer 11 may be provided on the second surface 3, said layer 11 covering the second surface 3 and whereby the layer 11 is resistant to a dry etching process. By patterning the layer 11 in such a way as to create openings in the layer, whereby an opening preferably comprises the area resulting from the orthogonal projection of the electrode 4 on the second surface 3, a first etch mask pattern 8 is fabricated.

Preferably the first and second etch mask patterns can comprise any material capable of blocking the dry-etching, in selected areas of the substrate; typically photoresist can be used as an etch mask pattern. Photoresist can be any photo-sensitive material used in photolithography that can transfer a pattern from the mask onto the substrate.

Several dry-etching techniques can be applied for fabricating the probe according to any embodiments of the invention, for instance Xenon difluoride etching, reactive ion etching or deep reactive ion etching. Preferably deep reactive ion etching is used to etch away parts of the substrate 1. Advantageously, by using this etching process, etch depths of hundreds of micrometers are achieved with almost vertical sidewalls. The primary technology is based on the so-called patented "Bosch process" (FR 2948495). The process can easily be used to etch completely through a silicon substrate, and etch rates are 3-6 times higher than wet etching.

In some other embodiments the method may further comprise after step (a2(ii)), a step (a2(iii)) whereby the first etch mask pattern 10 is removed without removing the second etch mask pattern 8, and then in step (a2(iv)) dry-etching of the second surface 3 is performed perpendicularly (arrow 9 in Fig. 2) to the second surface 3. As a result of this step the substrate is thinned but not eliminated, thereby defining the thickness 13 of said horizontal part 31 in said T-shaped cross-section 32. As a step (a3), the portion of the substrate lying within the second mask 8 is at least partially cut out from the substrate 1, thereby providing said probe (if entirely cut out) or provide said probe still attached via attachment points to the rest of the substrate 1 (if partially cut out).

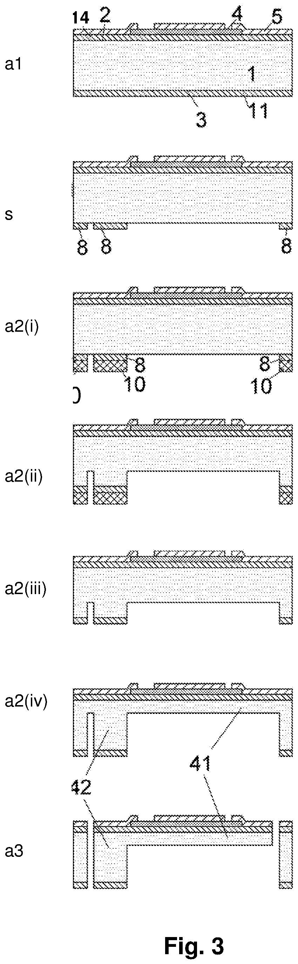

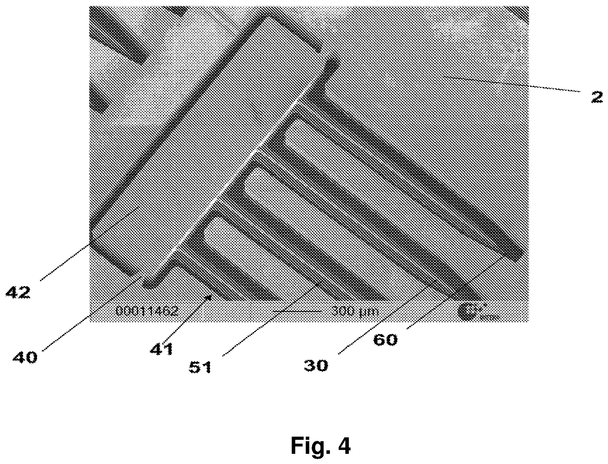

Preferably, the partial cutting of the probe can be achieved by dry-etching the first surface 2 of the substrate 1 after patterning of an isolation layer 14 and passivation layer 5 on the first surface 2 of the substrate 1. In Fig. 3 the partial cutting is illustrated in a clear way in steps (a2(iv)) where the connector part 42 is attached to the substrate 1 via an attachment point 40. Step (a3) is performed either everywhere or not everywhere (via appropriate masking of the first surface) in order to maintain attachment points 40. In Fig. 4 the partial cutting of the probe is illustrated as seen from the second surface of the substrate. Preferably at least one attachment point 40 between the connector part 42 and the substrate 1 remains after the partial etching and thus cutting of the first surface. Preferably the attachment point 40 is easily released from the rest of the substrate. For instance, using tweezers to release the probe from the substrate can be easily performed. Advantageously as a result, no additional dicing is required to separate the probes fro the fabrication wafer.

An advantage of a method in accordance with embodiments of the present invention is that it offers a solution for drastically reducing the cross-sectional area of a probe without compromising the resistance of the probe to bending, buckling or flexing. As a result of this drastically reduced cross-section the amount of damage the probe inflicts to the biological tissue is as low as possible. However, when making the probes too thin other problems such as increased fragility, flexing or buckling can occur during the insertion phase of the probe. Advantageously, the T-shaped cross-section additionally provides reinforcement of the probe in order to overcome these problems.

A probe 32 for brain recording or stimulation, according to one embodiment of the invention, comprises a T-shaped profile and the T-shape is defined by a horizontal part 31 and a vertical part 30, wherein the horizontal part has a first surface 50 and a second surface 51, wherein said vertical part 30 is attached to said second surface 51, and wherein said first surface 50 comprises one or more electrodes (recording/ stimulating sites) 4.

Preferably the probe according to any embodiments has a sharp distal end and has a stiffness that is sufficient for allowing insertion of the probe into a brain. More specifically the probe can be inserted in the brain without deflection.

Another embodiment of the present invention relates to a method of fabricating a probe for brain recording or stimulation, whereby the probe has shanks having a H-shaped cross-section defined by a vertical part and two horizontal parts. The method comprises the steps of attaching the free extremity of the vertical part of a shank according to anyone of the previous embodiments with either a further horizontal part or the free extremity of a vertical part of a second shaft according to anyone of the previous embodiments.

An advantage of using a probe with H-shaped cross-section shanks is that it doubles the amount of electrodes that can be used: the electrodes on the first surface of the first and second horizontal parts of the H-shaped cross-sectioned shanks.

Preferable the attaching of the free extremities of the vertical part of both shanks can be enabled by glue or anodic bonding. Any glue suitable for attaching electronic components can be used. Before applying the glue the free extremities are preferably cleaned.

Each figure is hereafter described in more details.

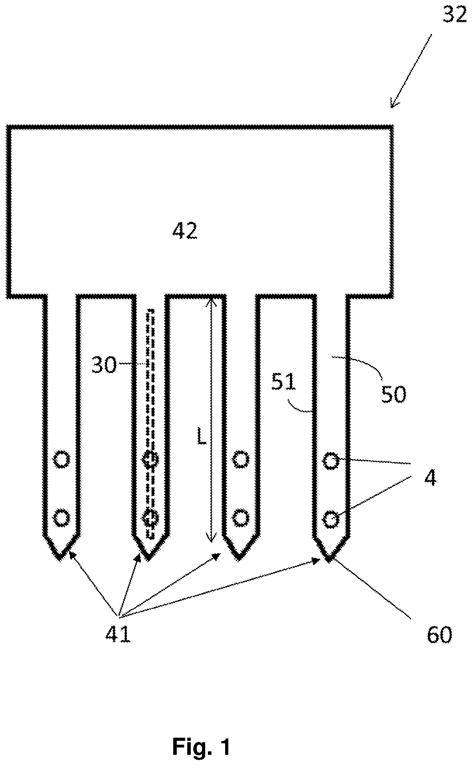

Figure 1 schematically illustrates a probe 32 for brain recording and/or stimulation according to an embodiment of the present invention. It comprises four shanks 41 and a connector part 42. From the connector part 42, each shank 41 protrudes along a same orientation. The shank 41 comprises a first side 50 and a second side 51 opposite to the first side. The first side 50 of the shank 41 comprises a number of recording and/or stimulating sites 4 that are connected to a signal processing circuitry. The second side 51 of the shank comprises a fin 30, protruding perpendicularly from the second side 51 and running on a major part of the shank 41 along the same orientation as the shank. The thickness of the elongate shaft portions and the triangular chisel-shaped tip portion is essentially constant. The probe has a first and second flat surface, the thickness of the probes extending between said surfaces. When the probe 32 is inserted in the brain, the shanks 41 penetrate the brain tissue. The penetrating of the shanks 41 is aided by a sharpened distal end 60 of the shank. This distal end 60 of the shank 41 is the furthermost point of the shank in relation to the point of attachment with the connector part 42.

Figure 2 is a schematic representation of a manufacturing method viewed orthogonally to the probe shank 41 through one recording and/or stimulating site 4, according to an embodiment of the present invention. The method comprises a step (a1) of providing a substrate 1 having a first 2 and a second 3 surface, whereby the first surface 2 comprises at least one recording and/or stimulating site 4. In a next step (a2(i)) a first etch mask pattern 10 is provided on the second surface 3, and the first etch mask pattern 10 masks the area corresponding to the fin 30 of the shank 41. In step (a2(ii)) dry-etching of the second surface 3 is performed in a direction 9 perpendicular to the second surface 3 in such a way as thinning, but not eliminating, at least part of said substrate 1 away from the first etch mask pattern 10, thereby defining the height 12 of the fin 30 of the shank 41. The first etch mask pattern 10 is resistant to the dry-etching process performed in step (a2(iii)).

Preferably after the recording and/or stimulating sites 4 are defined a passivation layer 5 is provided before performing the first step (a1). Preferably, an etch mask layer 11 and an isolation layer 14 on respectively the second surface 3 and the first surface 2 of the substrate 1 can be applied. The substrate material used is preferably a semiconductor and more specifically silicon. The recording and/or stimulating sites 4 can be deposited using several deposition techniques known in the prior art, such as for example electron beam deposition, sputtering or plasma vapour deposition or any other conventional thin film deposition technique.

In another embodiment, the method may further comprise a step (s) after step (a1) and before step (a2(ii)), whereby a second etch mask pattern 8 is provided on the second surface 3, delimiting the shape of the shanks 41 of the probe 32 to be fabricated. The second etch mask pattern 8 can be created by patterning the etch mask layer 11 covering the second surface 3 in such a way as to create an opening 7 in the layer, whereby the opening 7 preferably comprises the masking area corresponding to the fin 30 of the probe 32. Preferably step (s) is performed before step (a2(i)). Preferably at least part of the masking area 8 corresponds to the connector part 42 of the probe 32. The second etch mask pattern 8 is resistant to a dry-etching process.

Several dry-etching techniques can be applied for fabricating the probe according to any embodiments of the invention, for instance Xenon difluoride etching, reactive ion etching or deep reactive ion etching. Preferably deep reactive ion etching is used to etch away parts of the substrate 1. Advantageously, by using this etching process, etch depths of hundreds of micrometers are achieved with almost vertical sidewalls. The primary technology is based on the so-called patented "Bosch process" (FR 2948495). The process can easily be used to etch completely through a silicon substrate, and etch rates are 3-6 times higher than wet etching.

In some other embodiments the method further can comprise after step (a2(ii)), a step (a2(iii)) whereby the first etch mask pattern 10 is removed without removing the second etch mask pattern 8, and then in step ((a2(iv)) dry-etching of the second surface 3 is performed in a direction perpendicular 9 to the second surface 3. As a result of this step the substrate is thinned but not eliminated, thereby defining the thickness 13 of the horizontal part 31 of the shank 41 having T-shaped cross-section, and thus also defining the thickness of the shank 41. As a final step (a3) the probe 32 is partially cut out the substrate 1, thereby providing said probe 32. Preferably, the partial cutting of the probe 32 can be achieved by dry-etching the first surface 2 of the substrate 1 after patterning of an isolation layer 14 and the passivation layer 5 on the first surface 2 of the substrate 1. In Fig. 3 the partial cutting is illustrated in a clear way in steps (a2(iv)) and (a3). In step (a2(iv)) the connector part 42 is attached to the substrate and the shanks 41 are fabricated. The cutting is performed in step (a3) and looking along the probe shank 41 the probe is completely cut out. Moreover, in Figure 4 the partial cutting of the probe is illustrated as seen from the second surface 3 of the substrate 1. Preferably at least one attachment point 40 between the connector part 42 and the substrate 1 remains after the partial etching and thus cutting of the first surface. Preferably the attachment point 40 is easily released from its coupled state. For instance, using tweezers to release the probe from the substrate can be easily performed.

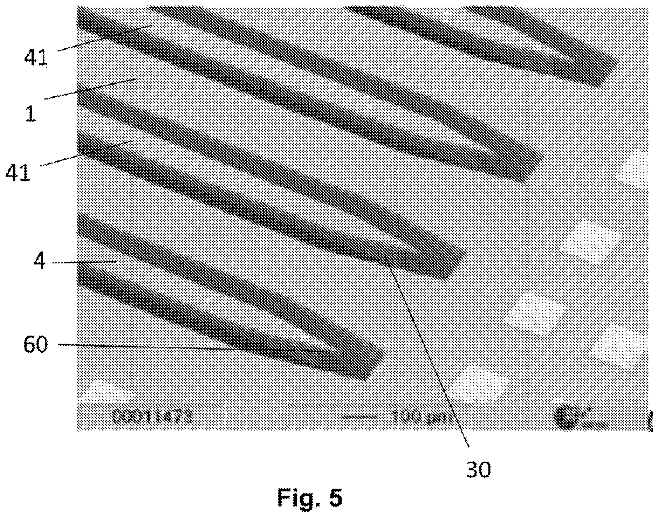

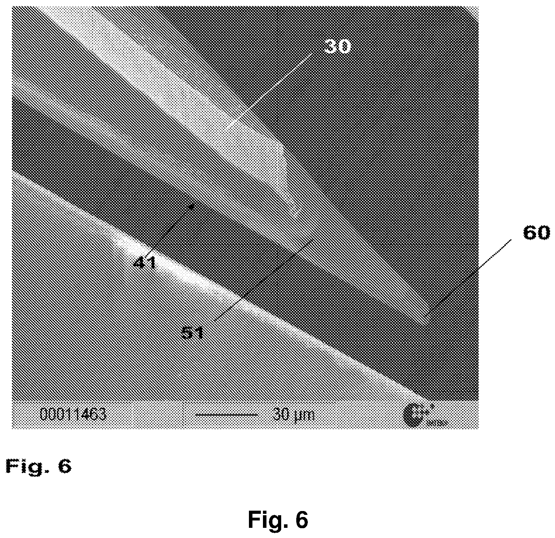

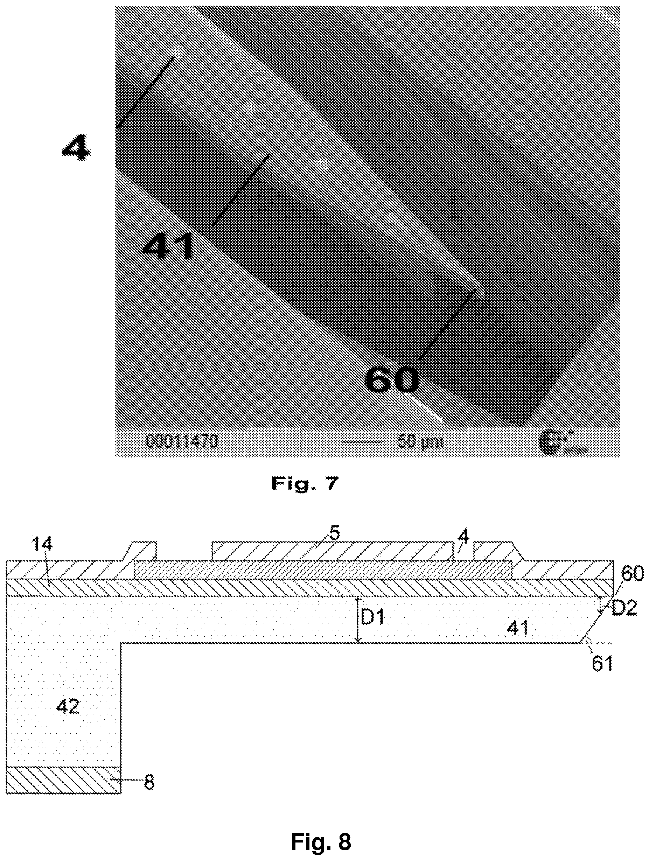

Figure 5 illustrates the shanks 41 have a T-shaped cross-section, whereby the fin 30 defines the vertical part of the T-shaped cross-section. The distal end 60 of the shanks 41 are preferably sharpen as an additional step (b) after step (a3) according to some embodiments of the invention. Fig. 6 shows an enlarged picture of the fin 30 of a shank 41 seen on second side 51 of the shank. Fig. 7 shows an enlarged picture of the distal end 60 of a shank 41 comprising recording and/or stimulating sites 4.

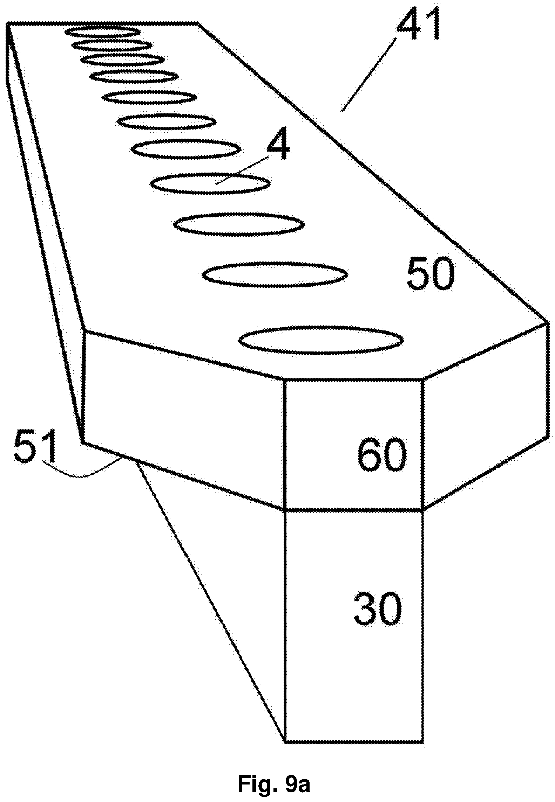

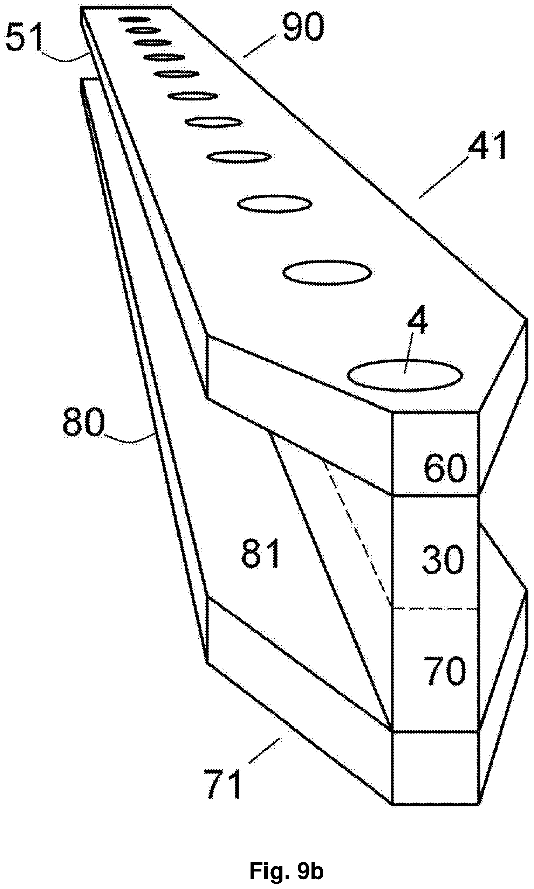

Fig. 9a is a schematic representation of a T-shaped probe shank 41 and Fig. 9b offers a schematic representation of an H-shaped probe shank 90. The H-shaped probe shank 90 is constructed by attaching the free extremity of the fin 30 of a shank 41 of a first probe to the fin 70 of a shank 71 of a second probe having a first side 80 and second side 81 that is opposed to the first side. The first side 80 of the second shank 71 comprises at least one recording and/or stimulating sites (not shown). In other embodiments of the invention, an H-shaped probe shank 90 can be constructed by attaching the free extremity of the fin 30 of a shank 41 directly to the second side 81 of a second shank 71 comprising no fin 70, whereby the first side of this second shank 71 may comprise recording and/or stimulating sites 4.

Preferably the sharpening of the distal end 60 can be performed by grinding the second side 51 of the shank. As a result of this sharpening, the height of the horizontal part 13 of the T-shaped cross-section is not uniform throughout the shank length L. This is schematically illustrated in Fig. 8.The thickness D2 of the horizontal part of the T-shaped cross-section 31 at the distal end 60 is smaller than the thickness D1 of the horizontal part of the T-shaped cross-section at a point on the shank 41 away from the distal end 60. More specifically, the second side 51 of the shank 41 comprising the distal end 60 makes an angle 61 with the second side of the shank 41 away from the distal end 60. This angle is smaller than 90° and is preferably smaller than 65°. The tip present at the distal end 60 lies in the plane defined by the probe's first side.

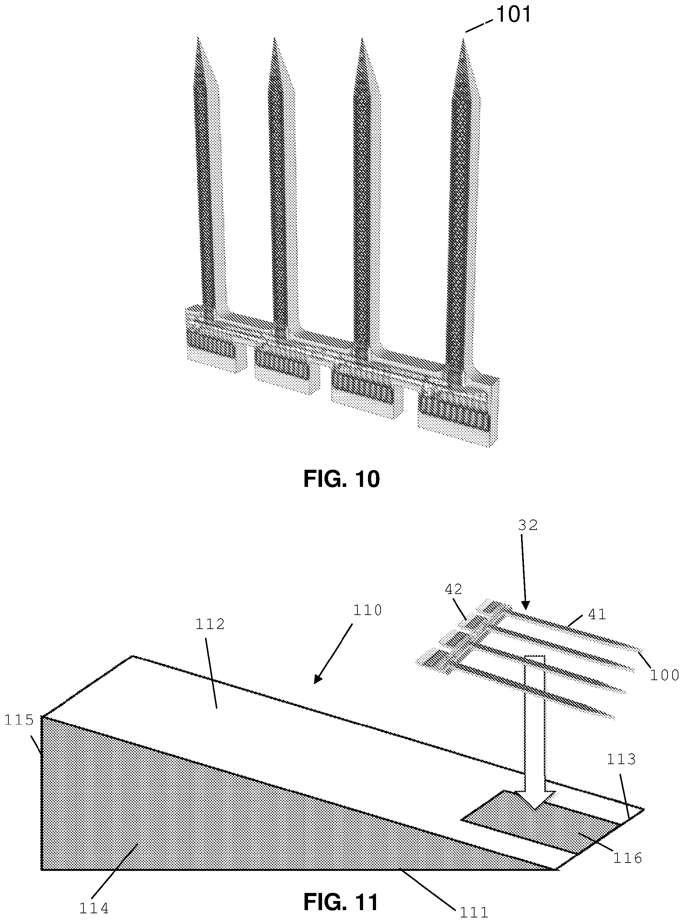

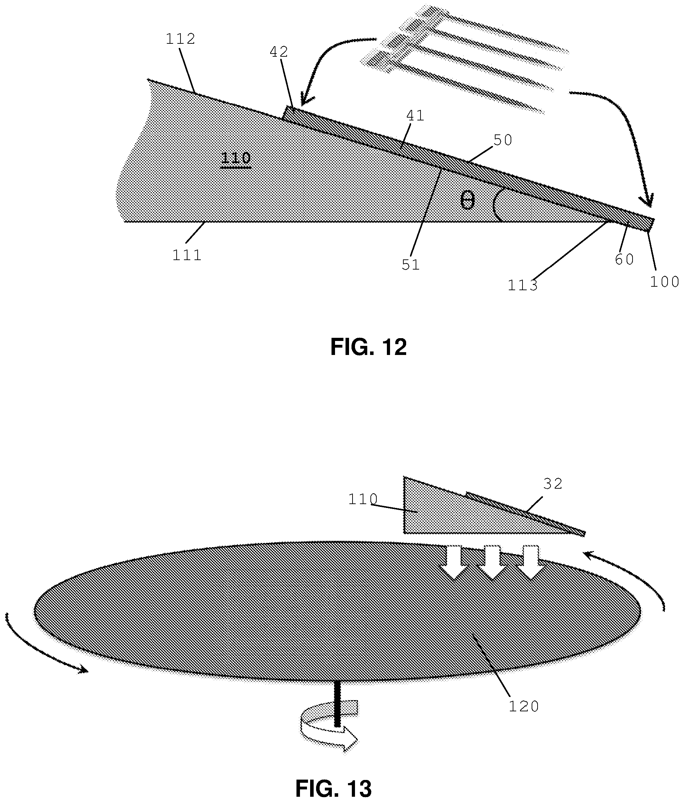

The grinding of the distal end 60 can be performed by temporary fixing the unsharpened probe 32 on a carrier 110 (see Fig. 11), which is sloped under an angle, in such a way that the second side 51 of the shank 41 comprising the distal end of the probe 32 is leading over the carrier 110. The carrier 110 then can be positioned on a rotating polishing disk 120 (see Fig. 13) which grinds the second side 51 of the shank 41 comprising the distal end 60. The carrier 110 with probe 32 can be positioned on a polishing table e.g. for several minutes resulting in a sharp pointy tip. In this example, the above mentioned angle 61 is determined by the angle of the sloped carrier 110 used for grinding. Other methods can also be used for the sharpening of the distal end, for instance by using anisotropic etching techniques that can etch the silicon planes of the probe tip. Also selective etching techniques can be used which apply a high dopant implantation onto the silicon.

Figure 10 shows a neural probe obtainable by the method of the invention. Each shank has a sharp pointed tip 101, able to pierce through the Dura without the dimpling effect. The fin is not represented but is present.

Preferred embodiments of the method of producing such a probe are illustrated in figures 11-15. The method as described hereafter is also applicable to a single shaft probe. Referring first to figure 11, a probe 32 is provided having an essentially constant thickness of the elongate shank 41 and the triangular chisel-shaped tip portions at the distal end, i.e. the probe has a first and second flat side 50/51, the thickness of the shanks extending between said sides. The probe is temporarily attached (e.g. by its fin(s)) at said second side 51, to a carrier 110, which is preferably a solid carrier block, e.g. made of aluminum. The carrier block shown in figure 11 is wedge-shaped, with a flat base surface 111 and a slanted top surface 112, forming a sharp angle with respect to the base surface, so that a straight edge 113 is formed between the base and top surfaces 111 and 112. The carrier 110 further comprises parallel side walls 114 and a back wall 115 perpendicular to the base surface. Other outer shapes of the carrier are possible, as long as the carrier comprises a first and second surface 111/112 forming a wedge, i.e. being oriented at a sharp angle with respect to each other, and joined by an edge 113 of the carrier.

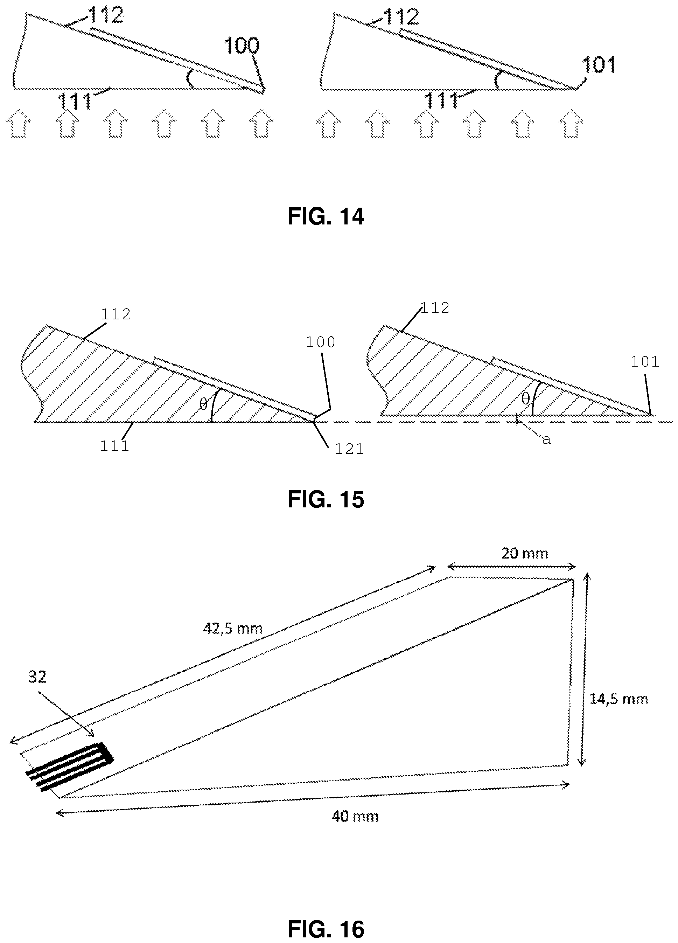

As seen in figures 11 and 12, the probe 32 is releasably attached to a contact location 116 of the carrier's slanted top surface 112, with at least part of the triangular tip portions at the distal end 60 extending over the edge 113, all triangular tip portions being placed at the same distance from the edge 113. This means that a portion of the shanks extends downward from the base surface 111 when said base surface is placed horizontally (see figure 12). In the case shown in figure 12, the connector part 42 and shanks 41 of the probe 32 are removably attached to the top surface 112 of the carrier block 110 with a suitable temporary adhesive, e.g. a resin or a wax. The contact location 116 on the slanted surface 112 of the block may be provided with an alignment structure, preferably comprising a set of grooves oriented perpendicularly to the edge 113, and at a mutual distance corresponding to the mutual distance of the probes in the array, so that the probes fit into said grooves. More preferably, the alignment structure is a negative imprint of the entire probe array, including the connector part 42, so that the entire probe 32 can be fitted into the carrier block's top surface 112. The above named adhesive is preferably used in addition to the alignment structure. Extending outward from the carrier edge 113 is the straight edge 100 of the chisel-shaped triangular probe tips at the distal end 60. The straight edge 100 is essentially perpendicular to the surface 112 of the carrier.
Then the carrier block/probe combination is subjected to a grinding step as illustrated in figure 13. Preferably a known grinding/polishing tool is used, having a rotating grinding disc 120 onto which the carrier block 110 can be placed with a suitable pressure. The carrier block can be fixed onto a micro-drive which lowers the carrier onto the rotating grinding/polishing disk. Suitable grinding/polishing tools that can be used are the Saphir 320/330 series from ATM. In the case shown in figure 12, the carrier material, the material of the grinding surface, the applied pressure and grinding time are chosen so that substantially no or very little material is removed from the carrier block's base surface 111 during said grinding step, while all the material of the probe tips extending below the base surface 111 is removed during said step. The result of the grinding step is shown in figure 14. After a suitable grinding time, the portion of the probes extending below the base surface 111 is removed, and a sharp tip 101 is formed at the distal end of the probes. The angle θ of the carrier block 110 is thereby substantially transferred to the probe tips.

Another embodiment is illustrated in figure 15. In this case, the material of the carrier 110, the material of the grinding surface, the grinding pressure and time are chosen so that the carrier block's base surface 111 is thinned during the grinding step, together with the shank tip. The shanks are attached to the carrier block without the triangular tip portions at the distal end 60 extending over the edge 113, preferably with the lower end 121 of the tip placed on the edge 113, as shown in figure 15. During the grinding step, a thickness 'a' of the carrier block and of the tip material is simultaneously removed, leading also to a sharp tip 101 at the end of the grinding step, with the angle θ of the carrier block transferred to the shank tip. In both embodiments shown in figures 14 and 15, the tip 101 lies in the plane defined by the probe's first side 50.

Variants of the above-described embodiments are included in the scope of the appended claims. The edge 113 may be curved instead of straight. This allows the formation of shank tips of different shape and size in one grinding step. Likewise, instead of placing the shanks of a probe at the same distance to a straight edge 113, it is possible to place the probe at an angle to the edge. Instead of an array of shanks, a single shank may be sharpened by the method of the invention. The angle θ is a sharp angle, i.e. lower than 90°, but preferably lower than 45°.

The carrier 110 is preferably but not necessarily a solid block. The grinding step can be followed by a polishing step, which may release the stresses induced by the grinding process. The polishing step can be performed in the same tool as the grinding step, e.g. by applying another polishing disk onto the rotating table of a rotatable grinding tool.

Instead of grinding only from the backside of the shank or probe, resulting in a pointed tip in the first side 50 of the probe or probe array, it is also possible to grind from both sides resulting in a pointed tip located in between the first and second side 50/51, and preferably at the centre of the shaft's thickness. This can be done by grinding the probe in two steps : in the first step, the probe is attached by its fin 30 (not shown) to the carrier block 110 as described above and a portion of the tip is removed by grinding and possibly polishing, preferably until at least about half of the straight edge 100 is removed. Then the probe is removed, reversed and re-attached by its first side 50, and a second grinding and possibly polishing step is done until a sharp tip is obtained, said tip lying in between the first and second side 50/51 of the probe. Both steps can be done by the method of the invention according to the embodiment of figure 14 or 15.

The sharpness of the carrier's edge 113 is not a crucial parameter. It is possible to grind the tip of the probe while the carrier contains a blunt edge 113. Since the angle of the carrier 110 and the thickness of the tip is known, one can calculate how far the chisel shaped tip at the distal end should extend from the edge 113.

### Example:

### Example (making reference to figures 2 and 3):

In a first step, an insulating layer (14, 11) is deposited on both wafer sides (2, 3). The layer (14, 11) is a stress compensated stack of a thermal silicon dioxide (SiO₂) grown at 950 °C, a silicon nitride (Si₃N₄) produced by low-pressure chemical vapor deposition (LPCVD) at 770 °C and a LPCVD low temperature oxide (LTO) deposited at 425 °C. The corresponding thicknesses are 200 nm, 100 nm and 200 nm. The stress compensation of the different layer stacks is based on residual stress values.

The electrode metallization 4 consists of 30 nm titanium (Ti), 200 nm gold (Au), 100 nm platinum (Pt) and 30 nm Ti. These layers are deposited by evaporation and patterned using a lift-off process. The evaporation of the metals was performed in a static deposition mode with the wafer position fixed above the evaporation source. By this choice frayed metal edges are avoided. In contrast, in the alternative dynamic mode, where up to six wafers are rotated above crucibles, negatively tapered resist sidewalls are partly covered due to an increased angle of incidence. While titanium serves as the adhesion layer to the dielectric thin films, gold is used to minimize the interconnection resistance and platinum is the electrode material of choice. The metal stack is covered by a 1.5 µm thick passivation multilayer 5. This is again a stress compensated layer stack of alternating PECVD SiOₓ (210 nm) and SixNy (160 nm) thin films. The passivation is patterned by reactive ion etching (RIE) using the STS RIE Multiplex etcher of Surface Technology Systems, UK, thus opening electrodes and contact pads, as indicated in figure 2 and 3, step a1. A RIE recipe optimized for the etching of SiO₂ with a platen power of 219 W; a process pressure of 100 mTorr; and gas flows of 15 sccm, 35 sccm and 50 sccm, for tetrafluoromethane (CF₄), fluoroform (CHF₃) and argon, respectively, is applied. As the dry etching stops at the Ti surface, Pt is protected against the CF₄/CHF₃ plasma.

A subsequent wet etching of the upper Ti on the electrodes and contact pads in 1% hydrofluoric acid (HF) exposes the Pt electrodes.

The rear SiOx/SixNy etch masks 11 are patterned using RIE, as shown in figure 2 and 3 (s). A photoresist layer 10 is spin coated on the rear of the wafer to define the step of the T-shape, as shown in figure 2 a2(i). The first DRIE step using an STS ICP Multiplexetcher from Surface Technology Systems, UK etches the T-shape step, as shown in figure 2 a2(ii). Next, the photoresist 10 is removed, as shown in figure 2 and 3 a2(iii) and the second DRIE 9 is applied, which defines the overall geometry of the shaft thickness (also using an STS ICP Multiplexetcher from Surface Technology Systems, UK), see figure a2 (iv).

The final DRIE step 9 is performed from the wafer front prior front side patterning of the SiOx/SixNy passivation layer 5 (using RIE) to complete the release of the probes, as shown in figure 2 and 3 (a3). The fabrication wafer with probes suspended by a pair of thin struts is finally separated from the support wafer.

It is to be understood that the invention is not limited to the particular features of the means and/or the process steps of the methods described as such means and methods may vary. It is also to be understood that the terminology used herein is for purposes of describing particular embodiments only, and is not intended to be limiting. It is also to be understood that plural forms include singular and/or plural referents unless the context clearly dictates otherwise. It is moreover to be understood that, in case parameter ranges are given which are delimited by numeric values, the ranges are deemed to include these limitation values.

The particular combinations of elements and features in the above detailed embodiments are exemplary only. As those skilled in the art will recognize, variations, modifications, and other implementations of what is described herein can occur to those of ordinary skill in the art without departing from the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

## Claims

1. A probe (32) for brain recording and/or stimulation, said probe (32) comprising one or more shanks (41) having a same orientation, wherein each of said shanks (41) has a first side (50) and a second side (51) opposed to said first side (50) and comprises at least one recording and/or stimulating site (4) on said first side (50), wherein each of said shanks (41) further comprises a fin (30) protruding perpendicularly from said second side (51) and running on at least a part of the length of said shank (41) along said same orientation, wherein each of said shanks (41) has a sharp pointed tip at its distal end (60), **characterised in** said tip lying in the plane defined by said first side (50).

2. The probe (32) according to claim 1, wherein said shanks have a T-shaped cross-section provided by said fin (30) protruding perpendicularly from said second side (51).

3. The probe (32) according to claim 1 or 2, wherein said first side (50) has a same orientation at the distal end (60) of the shank (41) and away from said distal end (60) while said second side (51) at said distal end (60) makes an angle (61) with said second side (51) away from said distal end (60).

4. The probe (32) according to any one of the preceding claims, wherein said tip has a triangular shape.

5. The probe (32) according to any one of the preceding claims, wherein said tip has a chisel shape.

6. A probe (32) according to any of the previous claims, further comprising at least one second shank (71), wherein said fin (30) is attached to either:
a) a fin (70) of said second shank (71), said second shank (71) having a first side (80) and a second side (81) opposed to said first side (80) and comprising at least one recording and/or stimulating site on said first side (80) and said fin (70) protruding perpendicularly from said second side (81) and running on at least a part of the length of said second shank (71) along said same orientation, or
b) the second side (81) of said second shank (71) having a first side (80) and a second side (81) opposed to said first side (80) and comprising at least one recording and/or stimulating site (4) on said first side (80), or
c) the second side (81) of said second shank (71) having a first side (80) and a second side (81) opposed to said first side (80).

7. A method for manufacturing a probe (32) for brain recording and/or stimulation, said probe (32) comprising one or more shanks (41) having a same orientation according to any of claims 1 to 6, the method comprising the steps of: providing a probe (32) for brain recording and/or stimulation, said probe (32) comprising one or more shanks (41) having a same orientation, wherein each of said shanks (41) has a first side (50) and a second side (51) opposed to said first side (50) and comprises at least one recording and/or stimulating site (4) on said first side (50) and a fin (30) protruding perpendicularly from said second side (51) and running on at least a part of the length of said shank (41) along said same orientation; and wherein providing said probe comprises the steps of:
a1) providing a substrate (1) having a first (2) and a second (3) surface opposed to said first surface (2), said first surface (2) comprising said at least one recording and/or stimulating site (4) of each of said one or more shanks (41) of the probe (32) to be manufactured;
a2) (i) providing a first etch mask pattern (10) on said second surface (3), said first etch mask pattern (10) masking the area corresponding to the fin (30) of each of said one or more shanks (41) of the probe (32) to be manufactured, said first etch mask pattern (10) being resistant to a dry-etching process, and
ii) dry-etching said second surface (3) with a dry-etching process for which said first etch mask pattern (10) is resistant, in a direction (9) perpendicular to said second surface (3) in such a way as thinning, but not eliminating, at least part of said substrate (1) away from said first etch mask pattern (10), thereby defining the height of said fins (30),
b) sharpening a distal end (60) of said shanks (41) in such a way as to provide said shanks with a sharp pointed tip at its distal end (60), **characterised in** said tip lying in the plane defined by said first side (50).

8. The method according to claims 7, wherein the obtained probe (32) has shank(s) (41) with a T-shaped cross-section.

9. The method according to claim 7 or 8, wherein said probe (32) further comprises a connector part (42) from which said one or more shanks (41) protrude along said same orientation and wherein in step (a2 (i)) said first etch mask pattern (10) is further masking the area corresponding to said connector part (42) of the probe (32) to be manufactured.

10. The method according to any of claims 7 to 9, wherein said first etch mask pattern (10) is masking exactly the area corresponding to the fin (30) of each of said one or more shanks (41) of the probe (32) to be manufactured.

11. The method according to any one of claims 7 to 10, further comprising a step (s) after step (al) and before step (a2 (ii)), of providing on said second surface (3), a second etch mask pattern (8) delimiting the shape of the shanks (41) of the probe (32) to be fabricated, said second etch mask pattern (8) being resistant to a dry etching process.

12. The method according to claim 11, wherein said step (s) is performed before step (a2(i)).

## Patentansprüche

1. Sonde (32) zur Hirnaufzeichnung und/oder -stimulation, wobei die Sonde (32) einen oder mehrere Schenkel (41) umfasst, welche die gleiche Ausrichtung aufweisen, wobei jeder der Schenkel (41) eine erste Seite (50) und eine zweite, der ersten Seite (50) entgegengesetzte Seite (51) aufweist und an der ersten Seite (50) mindestens eine Aufzeichnungs- und/oder Stimulationsstelle (4) umfasst,
wobei jeder der Schenkel (41) ferner eine Rippe (30) umfasst, die senkrecht von der zweiten Seite (51) hervorsteht und auf mindestens einem Teil der Länge des Schenkels (41) in der gleichen Ausrichtung verläuft, wobei jeder der Schenkel (41) an seinem distalen Ende (60) eine scharf zulaufende Spitze aufweist,
**dadurch gekennzeichnet, dass** die Spitze in der Ebene liegt, die durch die erste Seite (50) definiert ist.

2. Sonde (32) nach Anspruch 1, wobei die Schenkel einen T-förmigen Querschnitt aufweisen, der durch die Rippe (30) bereitgestellt wird, die senkrecht von der zweiten Seite (51) hervorsteht.

3. Sonde (32) nach Anspruch 1 oder 2, wobei die erste Seite (50) an dem distalen Ende (60) des Schenkels (41) und weg von dem distalen Ende (60) eine gleiche Ausrichtung aufweist, wohingegen die zweite Seite (51) an dem distalen Ende (60) weg von dem distalen Ende (60) einen Winkel (61) zur zweiten Seite (51) beschreibt.

4. Sonde (32) nach einem der vorhergehenden Ansprüche, wobei die Spitze eine Dreieckform aufweist.

5. Sonde (32) nach einem der vorhergehenden Ansprüche, wobei die Spitze eine Meißelform aufweist.

6. Sonde (32) nach einem der vorhergehenden Ansprüche, ferner mindestens einen zweiten Schenkel (71) umfassend, wobei die Rippe (30) angebracht ist an entweder:
a) einer Rippe (70) des zweiten Schenkels (71), wobei der zweite Schenkel (71) eine erste Seite (80) und eine zweite, der ersten Seite (80) entgegengesetzte Seite (81) aufweist und an der ersten Seite (80) mindestens eine Aufzeichnungs- und/oder Stimulationsstelle umfasst und die Rippe (70) senkrecht von der zweiten Seite (81) hervorsteht und auf mindestens einem Teil der Länge des zweiten Schenkels (71) in der gleichen Ausrichtung verläuft, oder
b) der zweiten Seite (81) des zweiten Schenkels (71), der eine erste Seite (80) und eine zweite, der ersten Seite (80) entgegengesetzte Seite (81) aufweist und an der ersten Seite (80) mindestens eine Aufzeichnungs- und/oder Stimulationsstelle (4) umfasst, oder
c) der zweiten Seite (81) des zweiten Schenkels (71), der eine erste Seite (80) und eine zweite, der ersten Seite (80) entgegengesetzte Seite (81) aufweist.

7. Verfahren zur Herstellung einer Sonde (32) zur Hirnaufzeichnung und/oder -stimulation, wobei die Sonde (32) nach einem der Ansprüche 1 bis 6 einen oder mehrere Schenkel (41) umfasst, welche die gleiche Ausrichtung aufweisen, wobei das Verfahren folgende Schritte umfasst: Bereitstellen einer Sonde (32) zur Hirnaufzeichnung und/oder -stimulation, wobei die Sonde (32) einen oder mehrere Schenkel (41) umfasst, welche die gleiche Ausrichtung aufweisen, wobei jeder der Schenkel (41) eine erste Seite (50) und eine zweite, der ersten Seite (50) entgegengesetzte Seite (51) aufweist und an der ersten Seite (50) mindestens eine Aufzeichnungs- und/oder Stimulationsstelle (4) umfasst, und eine Rippe (30), die senkrecht von der zweiten Seite (51) hervorsteht und auf mindestens einem Teil der Länge des Schenkels (41) in der gleichen Richtung verläuft; und
wobei das Bereitstellen der Sonde folgende Schritte umfasst:
a1) Bereitstellen eines Substrats (1) mit einer ersten Oberfläche (2) und einer zweiten, der ersten Oberfläche (2) entgegengesetzten Oberfläche (3), wobei die erste Oberfläche (2) mindestens eine Aufzeichnungs- und/oder Stimulationsstelle (4) jedes des einen oder der mehreren Schenkel (41) der herzustellenden Sonde (32) umfasst,
a2) (i) Bereitstellen einer ersten Ätzmaskenstruktur (10) auf der zweiten Oberfläche (3), wobei die erste Ätzmaskenstruktur (10) den Bereich maskiert, welcher der Rippe (30) jedes des einen oder der mehreren Schenkel (41) der herzustellenden Sonde (32) entspricht, wobei die erste Ätzmaskenstruktur (10) beständig gegen einen Trockenätzprozess ist, und
(ii) Trockenätzen der zweiten Oberfläche (3) in einem Trockenätzprozess, gegen den die erste Ätzmaskenstruktur (10) beständig ist, in einer Richtung (9) senkrecht zur zweiten Oberfläche (3) derart, dass zumindest ein Teil des Substrats (1) weg von der ersten Ätzmaskenstruktur (10) ausgedünnt, aber nicht beseitigt wird, wodurch die Höhe der Rippen (30) definiert wird,
b) Schärfen eines distalen Endes (60) der Schenkel (41) derart, dass die Schenkel mit einer scharf zulaufenden Spitze an ihrem distalen Ende (60) bereitgestellt werden,
**dadurch gekennzeichnet, dass** die Spitze in der Ebene liegt, die durch die erste Seite (50) definiert wird.

8. Verfahren nach Anspruch 7, wobei die erzielte Sonde (32) (einen) Schenkel (41) mit T-förmigem Querschnitt aufweist.

9. Verfahren nach Anspruch 7 oder 8, wobei die Sonde (32) ferner einen Anschlussteil (42) umfasst, von dem aus der eine oder die mehreren Schenkel (41) in dergleichen Richtung hervorstehen und wobei in Schritt (a2 (i)) die erste Ätzmaskenstruktur (10) ferner den Bereich maskiert, der dem Anschlussteil (42) der herzustellenden Sonde (32) entspricht.

10. Verfahren nach einem der Ansprüche 7 bis 9, wobei die erste Ätzmaskenstruktur (10) genau den Bereich maskiert, welcher der Rippe (30) jedes des einen oder der mehreren Schenkel (41) der herzustellenden Sonde (32) entspricht.

11. Verfahren nach einem der Ansprüche 7 bis 10, nach Schritt (a1) und vor Schritt (a2 (ii)) ferner einen Schritt (s) des Bereitstellens einer zweiten Ätzmaskenstruktur (8) auf der zweiten Oberfläche (3) umfassend, welche die Form der Schenkel (41) der zu fertigenden Sonde (32) begrenzt, wobei die zweite Ätzmaskenstruktur (8) beständig gegen einen Trockenätzprozess ist.

12. Verfahren nach Anspruch 11, wobei der Schritt (s) vor Schritt (a2 (i)) ausgeführt wird.

## Revendications

1. Sonde (32) pour enregistrement et/ou stimulation cérébrale, ladite sonde (32) comprenant une ou plusieurs tiges (41) ayant une même orientation, chacune desdites tiges (41) ayant une première face (50) et une seconde face (51) opposée à ladite première face (50) et comprenant au moins un point d'enregistrement et/ou de stimulation (4) sur ladite première face (50),
chacune desdites tiges (41) comprenant en outre une ailette (30) dépassant perpendiculairement de ladite seconde face (51) et passant sur au moins une partie de la longueur de ladite tige (41) en suivant ladite même orientation, chacune desdites tiges (41) comportant une pointe aiguisée à son extrémité distale (60),
**caractérisée en ce que** ladite pointe se situe dans le plan défini par ladite première face (50).

2. Sonde (32) selon la revendication 1, dans laquelle lesdites tiges ont une section transversale en forme de T créée par ladite ailette (30) dépassant perpendiculairement de ladite seconde face (51).

3. Sonde (32) selon la revendication 1 ou 2, dans laquelle ladite première face (50) a la même orientation à l'extrémité distale (60) de la tige (41) et en s'éloignant de ladite extrémité distale (60), alors que ladite seconde face (51) décrit à ladite extrémité distale (60) un angle (61) avec ladite seconde face (51) détournée de ladite extrémité distale (60).

4. Sonde (32) selon l'une quelconque des revendications précédentes, dans laquelle ladite pointe a une forme triangulaire.

5. Sonde (32) selon l'une quelconque des revendications précédentes, dans laquelle ladite pointe a une forme de ciseau.

6. Sonde (32) selon l'une quelconque des revendications précédentes, comprenant en outre au moins une seconde tige (71), ladite ailette (30) étant fixée :
a) soit à une ailette (60) de ladite seconde tige (71), ladite seconde tige (71) ayant une première face (80) et une seconde face (81) opposée à ladite première face (80) et comprenant au moins un point d'enregistrement et/ou de stimulation sur ladite première face (80) et ladite ailette (70) dépassant perpendiculairement de ladite seconde face (81) et passant sur au moins une partie de la longueur de ladite seconde tige (71) en suivant ladite même orientation, ou
b) soit la seconde face (81) de ladite seconde tige (71) a une première face (80) et une seconde face (81) opposée à ladite première face (80) et comprenant au moins un point d'enregistrement et/ou de stimulation (4) sur ladite première face (80), ou
c) soit la seconde face (81) de ladite seconde tige (71) a une première face (80) et une seconde face (81) opposée à ladite première face (80).

7. Procédé de fabrication d'une sonde (32) pour enregistrement et/ou stimulation cérébrale, ladite sonde (32) comprenant une ou plusieurs tiges (41) ayant une même orientation selon l'une quelconque des revendications 1 à 6, ce procédé comprenant les étapes suivantes : prévision d'une sonde (32) pour enregistrement et/ou stimulation cérébrale, ladite sonde (32) comprenant une ou plusieurs tiges (41) ayant une même orientation, chacune desdites tiges (41) ayant une première face (50) et une seconde face (51) opposée à ladite première face (50) et comprenant au moins un point d'enregistrement et/ou de stimulation (4) sur ladite première face (50) et une ailette (30) dépassant perpendiculairement de ladite seconde face (51) et passant sur au moins une partie de la longueur de ladite tige (41) en suivant ladite même orientation , la prévision de ladite sonde comprenant les étapes suivantes :
a1) prévision d'un substrat (1) ayant une première (2) et une seconde (3) surface opposée à ladite première surface (2), ladite première surface (2) comprenant ledit au moins un point d'enregistrement et/ou de stimulation (4) de chacune desdites ou plusieurs tiges (41) de la sonde (32) à fabriquer ;
a2) (i) prévision d'un premier motif de masque de gravure (10) sur ladite seconde surface (3), ledit premier motif de masque de gravure (10) masquant les zones correspondant à l'ailette (30) de chacune desdites une ou plusieurs tiges (41) de la sonde (32) à fabriquer, ledit premier motif de masque de gravure (10) résistant à un processus de gravure sèche, et
(ii) gravure sèche de ladite seconde surface (3) par un processus de gravure sèche auquel ledit premier motif de masque de gravure (10) résiste, dans un sens (9) perpendiculaire à ladite seconde surface (3) de manière à amincir mais pas à éliminer au moins une partie dudit substrat (1) dudit premier motif de masque de gravure (10) en définissant ainsi la hauteur desdites ailette (30),
b) aiguisage d'une extrémité distale (60) desdites tiges (41) de manière à pourvoir lesdites tiges d'une pointe aiguisée à leur extrémité distale (60),
**caractérisé en ce que** ladite pointe se situe dans le plan défini par ladite première face (50).

8. Procédé selon la revendication 7, dans lequel la sonde obtenue (32) comporte une ou des tiges (41) ayant une section transversale en forme de T.

9. Procédé selon la revendication 7 ou 8, dans lequel ladite sonde (32) comprend en outre une pièce de connexion (42) de laquelle lesdites une ou plusieurs tiges (41) dépassent suivant ladite même orientation et dans lequel, dans l'étape (a2 (i)), ledit premier motif de masque de gravure (10) masque en outre la zone correspondant à ladite pièce de connexion (42) de la sonde (32) à fabriquer.

10. Procédé selon l'une quelconque des revendications 7 à 9, dans lequel ledit premier motif de masque de gravure (10) masque exactement la zone correspondant à l'ailette (30) de chacune desdites une ou plusieurs tiges (41) de la sonde (32) à fabriquer.

11. Procédé selon l'une quelconque des revendications 7 à 10, comprenant en outre une étape (s), après l'étape (a1) et avant l'étape (a2 (ii)), de prévision sur ladite seconde surface (3) d'un second motif de masque de gravure (8) délimitant la forme des tiges (41) de la sonde (32) à fabriquer, ledit second motif de masque de gravure (8) résistant à un processus de gravure sèche.

12. Procédé selon la revendication 11, dans lequel ladite étape (s) est réalisée avant l'étape (a2 (i)).
